# EUROPÄISCHE PATENTANMELDUNG

(11) **EP 0 632 027 A1**
(43) Veröffentlichungstag der Anmeldung: **04.01.1995**
(21) Anmeldenummer: 94109704.0
(22) Anmeldetag: 23.06.1994
(51) Int. Cl.: C07D 215/14

(54) **6-(Carbonyloxymethylene)-Chinoline**

(30) Priorität: 01.07.1993 DE 4321845
(71) Anmelder: BASF Aktiengesellschaft, D-67063 Ludwigshafen (DE)
(72) Erfinder: Pinkos, Rolf, Dr., D-67098 Bad Duerkheim (DE)

(57) **Zusammenfassung**

6-(Carbonyloxymethylene)-chinoline der allgemeinen Formel I 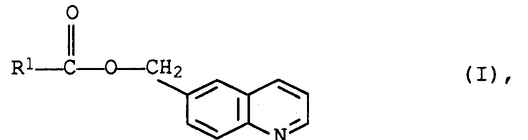
in der R¹ Wasserstoff, C₁- bis C₅-Alkyl, C₃- bis C₈-Cycloalkyl und Aryl, sowie ein Verfahren zu deren Herstellung und deren Umsetzung durch Hydrolyse zum 6-Hydroxymethylchinolin.

## Beschreibung

Diese Erfindung betrifft neue 6-(Methylenoxycarbonyl)-chinoline, ein verfahren zur Herstellung dieser 6-(Methylenoxycarbonyl)-chinoline durch Umsetzung von 6-Methylchinolin mit Perestern bei erhöhten Temperaturen und deren Hydrolyse zum 6-Hydroxymethylchinolin.

Aus J.A.C.S. 81, 5819 bis 5824 (1959) sind Umsetzungen von tert.-Butylpercarbonsäureestern insbesondere mit Olefinen bekannt. Ferner wird dort die Cu-I-katalysierte Reaktion von tert.-Butylpercarbonsäureestern mit einer Benzylwasserstoffatom enthaltenden Verbindungen beschrieben. Dabei tragen die Benzyleinheiten neben dem zu abstrahierenden H-Atom noch weitere Substituenten.

Aus Tetrahedron Lett. 21, 4917 bis 4920 (1980) ist das 6-(Methylchinolin)-2'-methylbenzoat bekannt, und auch dessen Hydrolyse zum 6-Hydroxymethylchinolin, jedoch ohne daß auf die Herstellung des Esters eingegangen wird.

Der vorliegenden Erfindung lag die Aufgabe zugrunde, einen einfachen Zugang zum 6-Hydroxymethylchinolin zu finden.

Demgemäß wurden neue 6-(Methylenoxycarbonyl)-chinoline der allgemeinen Formel I 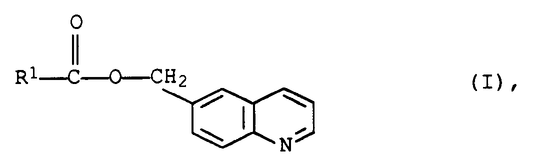
in der R¹ Wasserstoff, C₁- bis C₅-Alkyl, C₃- bis C₈-Cycloalkyl und Aryl bedeutet, sowie Verfahren zu deren Herstellung indem man 6-Methylchinolin mit Percarbonsäureestern der allgemeinen Formel II 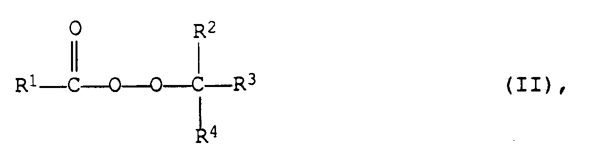
in der R¹ die oben genannten Bedeutungen hat und R², R³, R⁴ C₁- bis C₈-Alkyl, Aryl, C₃- bis C₈-Cycloalkyl bedeuten, in Gegenwart einer Kupfer-, Kobalt-, Nickel- und/oder Eisenverbindung bei Temperaturen von 60 bis 200°C umsetzt und ein Verfahren zur Umsetzung der 6-(Methylenoxycarbonyl)-chinoline mit einer Base bei Temperaturen von 40 bis 150°C durch Hydrolyse, gefunden.

Der Substituent R¹ der neuen 6-(Methylenoxycarbonyl)-chinoline I hat folgende Bedeutungen:
- Wasserstoff,
- C₁- bis C₅-Alkyl, bevorzugt C₁- bis C₃-Alkyl wie Methyl, Ethyl, n-Propyl und iso-Propyl,
- C₃- bis C₈-Cycloalkyl, bevorzugt C₅- bis C₈-Cycloalkyl wie Cyclopentyl und Cyclohexyl, besonders bevorzugt Cyclohexyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl.

Das erfindungsgemäße Verfahren läßt sich wie folgt durchführen:
Das 6-Methylchinolin und die Kupfer-, Kobalt-, Nickel- und/oder Eisenverbindung kann vorgelegt und in der Regel bei Temperaturen von 60 bis 200°C, bevorzugt 60 bis 140°C, besonders bevorzugt 70 bis 120°C und Drücken von 0,1 bis 3 bar, bevorzugt 0,5 bis 1,5 bar, besonders bevorzugt bei Normaldruck (Atmosphärendruck) vorgelegt und unter diesen Bedingungen die Percarbonsäureester II zugegeben werden. In der Regel sollte die Reaktionstemperatur möglichst niedrig gehalten werden, um die Nebenproduktbildung zu verringern.

Das Molverhältnis von Percarbonsäureester II zum 6-Methylchinolin beträgt in der Regel 0,01 : 1 bis 5 : 1, bevorzugt 0,05 : 1 bis 2 : 1, besonders bevorzugt 0,1 : 1 bis 1 : 1.

Als Kupfer-, Kobalt-, Nickel- und/oder Eisenverbindungen eignen sich Acetate, Sulfate, Phosphate, Halogenide und Carbonate, bevorzugt Kupfer-, Kobalt- und/oder Nickelverbindungen, besonders bevorzugt Cu-I- und Co-II-chloride oder -bromide.

Das Molverhältnis der Kupfer-, Kobalt-, Nickel- und/oder Eisenverbindung zum Perester beträgt in der Regel 0,001 : 1 bis 0,3 : 1, bevorzugt 0,01 : 1 bis 0,1 : 1.

Die Reaktion kann ohne Lösungsmittel, oder unter Zusatz von inerten Lösungsmitteln wie z.B. Benzol oder tert.-Butanol durchgeführt werden. Diese Lösungsmittel können im Gewichtsverhältnis von 0,01 : 1 bis 20 : 1, bevorzugt 0,02 : 1 bis 5 : 1, besonders bevorzugt 0,05 : 1 bis 1 : 1 zum 6-Methylchinolin eingesetzt werden. Ferner kann die Reaktion auch in Anwesenheit von C₁- bis C₁₀-Carbonsäuren, z.B. Essigsäure durchgeführt werden. Dadurch können auch die Ester der zugesetzten Carbonsäure erhalten werden.

Nach beendeter Reaktion können die entstandenen 6-(Methylenoxycarbonyl)-chinoline I direkt im Reaktionsgemisch, mit einer Base z.B. mit wäßrigem Natrium- oder Kaliumhydroxid hydrolysiert werden, oder nach Aufarbeitung (Reinigung) z.B. destillativer Abtrennung von Lösungsmittelresten und/oder nicht umgesetztem 6-Methylchinolin.

Die 6-(Methylenoxycarbonyl)-chinoline I können vor Hydrolyse auch rein isoliert werden, z.B. durch Destillation bei z.B. 0,1 mbar/150 bis 250°C oder Extraktion mit Lösungsmitteln wie z.B. Petrolether, Hexan, Heptan oder Ethern wie Methyl-tert.-butylether oder Diethylether oder Estern wie Ethylacetat, nachdem zuvor die im Reaktionsgemisch enthaltenden Carbonsäuren, z.B. durch Destillation entfernt oder Zugabe von Sodalösung, gebunden wurden.

Die Hydrolyse kann durch wäßrige Mineralsäure oder vorteilhafter durch wäßrige, z.B. 3 bis 30 %ige Alkali- oder Erdalkalilaugen erfolgen. Dabei werden 1 bis 2 Äquivalente des entsprechenden Hydroxids pro zuvor eingesetzten Percarbonsäureester, sofern das Reaktionsgemisch hydrolysiert werden soll, bzw. 1 bis 1,5 Äquivalente Hydroxid pro Mol 6-(Methylenoxycarbonyl)-chinoline I, sofern dieser rein eingesetzt wird, verwendet.

Die Hydrolysetemperatur beträgt in der Regel 20 bis 150°C, bevorzugt 40 bis 120°C, besonders bevorzugt 80 bis 110°C, wobei die Reaktion in der Regel nach 0,5 bis 3 Stunden beendet ist.

Werden die reinen 6-(Methylenoxycarbonyl)-chinoline I alkalisch verseift, so kann 6-Hydroxymethylchinolin durch Phasentrennung oder durch Extraktion des Reaktionsaustrages mit organischen Lösungsmitteln wie z.B. Ether, Benzol, Toluol, Ethylacetat oder Methylenchlorid und anschließende destillative Abtrennung des Lösungsmittels isoliert werden. Das 6-Hydroxymethylchinolin fällt gewöhnlich als Öl an, das alsbald kristallisiert.

Wird der Peresterreaktionsansatz direkt zur alkalischen Verseifung eingesetzt, so kann das 6-Hydroxymethylchinolin z.B. nach Abtrennen der organischen Phase, nach Abdestillieren von eventuell noch vorhandenem 6-Methylchinolin destillativ bei z.B. 0,1 bar/200 bis 250°C oder durch Kristallisation gewonnen werden. Vorteilhafter ist die Extraktion des Reaktionsaustrages mit unpolaren Lösungsmitteln wie Hexan oder Heptan. Dabei wird vorwiegend im Reaktionsaustrag enthaltenes 6-Methylchinolin zurückgewonnen. Durch Lösungsmittelwechsel auf Ether, wie z.B. Diethylether oder Methyl-tert.-butylether kann 6-Hydroxymethylchinolin extrahiert werden. Nach Abdestillieren des Ethers kristallisiert 6-Hydroxymethylchinolin.

Das 6-Methylchinolin kann z.B. nach der DE-A-609 383 aus p-Toluidin und Glycerin in Gegenwart von Schwefelsäure und Nitrobenzol hergestellt werden.

Die Substituenten R², R³ und R⁴ in den Verbindungen II haben folgende Bedeutungen:
- C₁- bis C₈-Alkyl, bevorzugt C₁- bis C₄-Alkyl wie Methyl, Ethyl, n-Propyl, iso-Propyl, n-Butyl, iso-Butyl, sec-Butyl und tert.-Butyl, besonders bevorzugt Methyl und Ethyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl und
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl.

Das 6-Hydroxymethylchinolin ist ein Zwischenprodukt bei der Synthese von Pharmaka (DE-A-35 09 618 und EP-A-381 375) und somit sind dies auch deren Edukte.

### Beispiele

### Beispiel 1

56 g 6-Methylchinolin und 0,4 g pulverförmiges CuBr wurden vorgelegt und auf 70°C erwärmt. Unter Rühren wurden 53 g tert.-Butylperbenzoat so zugegeben, daß die Reaktionstemperatur nicht über 100°C stieg. Nach beendeter Zugabe (ca. 30 Minuten) wurde der Reaktionsansatz noch 1 Stunde bei 100°C gehalten, dann auf ca. 50°C abgekühlt und mit 200 g Ethylacetat versetzt. Diese Mischung wurde mit 300 ml 10 %iger wäßriger Sodalösung extrahiert. Aus der organischen Phase wurde nach deren Abtrennung zuerst Ethylacetat, dann 35 g unumgesetztes Methylchinolin (80°C, 1 mbar) abdestilliert. Der verbleibende Rest wurde in 20 ml Ethylacetat aufgenommen und über Kieselgel (ca. 30 g) filtriert. Nach Abdestillieren des Ethylacetats kristallisierten 13,1 g (13 % Ausbeute. Der Schmelzpunkt des 6-Hydroxymethylchinolylbenzoates betrug 45°C.

### Beispiel 2

10 g 6-Hydroxymethylchinolylbenzoat und 18 g 10 %ige Natronlauge wurden 30 Minuten auf 100°C erhitzt. Nach Abkühlen wurde der Reaktionsansatz mit 20 ml Ethylacetat extrahiert und anschließend die organische Phase durch Abdestillieren des Lösungsmittels eingeengt. Es kristallisierten 5,7 g (95 % Ausbeute) 6-Hydroxymethylchinolin (Fp. 78°C, Lit. Kaslow et al., J.Org.Chem. 18, 55 (1953): 79 bis 80°C).

### Beispiel 3

Analog Beispiel 1 wurden 22 g 6-Methylchinolin, 0,2 g CuBr und 30 g Essigsäure mit 25 g tert.-Butylperbenzoat umgesetzt. Nach erfolgter Reaktion wurde der Reaktionsansatz noch 3 Stunden bei 100°C gehalten. Die Aufarbeitung erfolgte analog Beispiel 1, allerdings mit 400 ml 20 %iger Sodalösung. Durch Destillation wurden 15 g 6-Methylchinolin und 5 g 6-Hydroxymethylchinolylacetat (¹³C-NMR: 170,8; 150,7; 147,9; 136,2; 134,4; 129,9; 129,5; 128,0; 127,0; 121,5; 65,9; 21,0 ppm) erhalten. Im Kolonnensumpf (ca. 5 g) fand sich ein Gemisch aus überwiegend 6-Hydroxymethylchinolylacetat und -benzoat.

### Beispiel 4

150 g 6-Methylchinolin, 15 g tert.-Butanol und 1 g CuBr wurden vorgelegt und auf 80°C erwärmt. Danach wurden unter Rühren 81 g tert.-Butylperbenzoat so zugegeben, daß das Reaktionsgemisch gelinde siedete (95 bis 106°C). Nach beendeter Peresterzugabe (1 Stunde), wurde der Reaktionsansatz noch 30 Minuten bei 100°C gehalten, danach 18,5 g NaOH in 150 g Wasser zugegeben. Innerhalb 45 Minuten wurde tert.-Butanol bei einer Sumpftemperatur von 90 bis 100°C abdestilliert. Der Reaktionsansatz wurde auf 50°C abgekühlt und anschließend kontinuierlich zuerst mit n-Hexan, zur Extraktion des nicht umgesetzten 6-Methylchinolins, dann mit Methyl-tert.-butylether extrahiert. Aus dem Hexanextrakt erhielt man 118 g 6-Methylchinolin zurück, aus dem Methyl-tert.-butyletherextrakt wurden 15 g (9 % Ausbeute, 42 % Selektivität bezogen auf 6-Methylchinolin) kristallines 6-Hydroxymethylchinolin erhalten.

## Patentansprüche

1. 6-(Methylenoxycarbonyl)-chinoline der allgemeinen Formel I 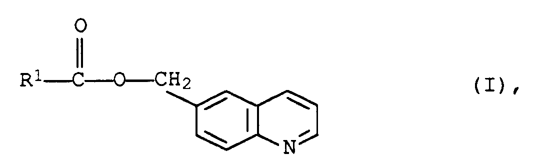 in der R¹ Wasserstoff, C₁- bis C₅-Alkyl, C₃- bis C₈-Cycloalkyl und Aryl bedeutet.

2. 6-(Methylenoxycarbonyl)-chinoline der Formel I nach Anspruch 1, in der R¹ Wasserstoff, C₁- bis C₃-Alkyl, C₅- bis C₈-Cycloalkyl, Phenyl, 1-Naphthyl und 2-Naphthyl bedeutet.

3. 6-(Methylenoxycarbonyl)-chinoline der Formel I nach Anspruch 1, in der R¹ Methyl oder Phenyl bedeutet.

4. Verfahren zur Herstellung von 6-(Methylenoxycarbonyl)-chinolinen der Formel I nach Anspruch 1, dadurch gekennzeichnet, daß man 6-Methylchinolin mit Percarbonsäureestern der allgemeinen Formel II 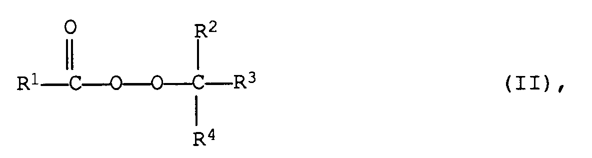 in der R¹ die oben genannten Bedeutungen hat und R², R³, R⁴ C₁- bis C₈-Alkyl, Aryl, C₃- bis C₈-Cycloalkyl bedeuten, in Gegenwart einer Kupfer-, Kobalt-, Nickel- und/oder Eisenverbindung bei Temperaturen von 60 bis 200°C umsetzt.

5. Verfahren zur Herstellung von 6-(Methylenoxycarbonyl)-chinolinen der Formel I nach Anspruch 1, dadurch gekennzeichnet, daß man 6-Methylchinolin mit Percarbonsäureestern der Formel II, in der R¹ Methyl oder Phenyl und R², R³, R⁴ Methyl bedeuten, in Gegenwart einer Kupfer-, Kobalt-, Nickel- und/oder Eisenverbindung bei Temperaturen von 60 bis 200°C umsetzt.

6. Verfahren zur Umsetzung von 6-(Methylenoxycarbonyl)-chinolinen der Formel I nach Anspruch 1, dadurch gekennzeichnet, daß man die 6-(Methylenoxycarbonyl)-chinoline I mit einer Base bei Temperaturen von 40 bis 150°C hydrolysiert.
